# EUROPEAN PATENT SPECIFICATION

(11) **EP 3 421 049 B1**
(45) Date of publication and mention of the grant of the patent: **29.07.2020**
(21) Application number: 16891597.3
(22) Date of filing: 26.10.2016
(51) Int. Cl.: A61K 9/08, A61K 31/216, A61K 47/02, A61K 47/12, A61K 47/14, A61P 17/00, A61K 45/00

(54) **LIQUID EXTERNAL PREPARATION**
FLÜSSIGES EXTERNES PRÄPARAT
PRÉPARATION EXTERNE LIQUIDE

(30) Priority: 25.02.2016 JP 2016034620
(43) Date of publication of application: 02.01.2019
(73) Proprietor: Hisamitsu Pharmaceutical Co., Inc., Tosu-shi, Saga 841-0017 (JP)
(72) Inventor: IMAMURA Kana, Tsukuba-shi Ibaraki 305-0856 (JP); FUJITA Naoko, Tsukuba-shi Ibaraki 305-0856 (JP); MICHINAKA Yasunari, Tsukuba-shi Ibaraki 305-0856 (JP)
(74) Representative: Grünecker Patent- und Rechtsanwälte PartG mbB
(86) International application number: PCT/JP2016/081771
(87) International publication number: WO 2017/145441

(56) References cited:
- EP-A1- 1 170 004
- AU-B2- 2012 216 593
- CN-A- 105 213 350
- JP-A- 2006 241 179
- US-A1- 2014 037 713

## Description

### Technical Field

The present invention relates to a liquid topical preparation.

### Background Art

Methods that involve administering a topical composition comprising an anticholinergic drug such as oxybutynin have been proposed as methods for treating hyperhidrosis (Patent Literature 1 and Patent Literature 2).

### Citation List

### Patent Literature

Patent Literature 1: U.S. Patent Application Publication No. 2014/0037713
Patent Literature 2: International Publication No. WO 2007/046102

### Summary of Invention

### Technical Problem

The present inventors have found the following new problem: the increased content of an anticholinergic drug in a liquid topical preparation results in "stickiness" derived from the anticholinergic drug, and tends to lower the comfortableness of the liquid topical preparation. Hence, an object of the present invention is to provide a liquid topical preparation having low "stickiness" even if it contains a high concentration of an anticholinergic drug.

### Solution to Problem

The present inventors have discovered that when a dicarboxylic acid ester is contained in a liquid topical preparation containing a high concentration of an anticholinergic drug, "stickiness" derived from the anticholinergic drug is suppressed, and thus have completed the present invention.

Specifically, the present invention provides a liquid topical preparation comprising water, an anticholinergic drug and a dicarboxylic acid ester, wherein the content of the anticholinergic drug ranges from 10 mass% to 20 mass% based on the total mass of the liquid topical preparation. The anticholinergic drug is oxybutynin or a pharmaceutically acceptable salt thereof. The content of the anticholinergic drug may range from 15 mass% to 20 mass% based on the total mass of the liquid topical preparation.

The dicarboxylic acid ester is one or more compounds selected from the group consisting of diisopropyl adipate, diethyl sebacate, diisopropyl sebacate, diisobutyl adipate, dimethyl succinate and dibutyl phthalate.

The mass ratio of the anticholinergic drug to the dicarboxylic acid ester may range from 1:0.25 to 1:0.75. The content of the dicarboxylic acid ester may range from 2.5 mass% to 15 mass% based on the total mass of the liquid topical preparation. The liquid topical preparation may further comprise one or more salts selected from the group consisting of lactate, tartrate, acetate and phosphate. The salt may be sodium lactate. The liquid topical preparation may be in a form of lotion. The liquid topical preparation may be for treating hyperhidrosis.

### Advantageous Effects of Invention

The liquid topical preparation of the present invention comprises a dicarboxylic acid ester, so as to suppress "stickiness" derived from an anticholinergic drug.

### Brief Description of Drawings

Figure 1 is a graph showing the results of a test for examining the influence of salts in lotions on the accumulation of oxybutynin in porcine hair follicles.
Figure 2 is a graph showing the results of a test for examining the influence of the concentrations of oxybutynin in lotions on the effect of suppressing sweating.
Figure 3 is a graph showing the results of a test for examining the influence of the concentrations of oxybutynin in lotions on the effect of suppressing sweating.
Figure 4 is a graph showing the results of a test for examining the influence of the concentrations of oxybutynin in lotions on the accumulation of oxybutynin in porcine hair follicles.

### Description of Embodiments

Hereinafter, the present invention will be described more specifically with reference to an embodiment.

One embodiment of the present invention is a liquid topical preparation comprising water, oxybutynin or a pharmaceutically acceptable salt thereof and a dicarboxylic acid ester being one or more compounds selected from the group consisting of diisopropyl adipate, diethyl sebacate, diisopropyl sebacate, diisobutyl adipate, dimethyl succinate and dibutyl phthalate, wherein the content of oxybutynin or a pharmaceutically acceptable salt thereof ranges from 10 mass% to 20 mass% based on the total mass of the liquid topical preparation. The liquid topical preparation can be used for treating hyperhidrosis.

Examples of anticholinergic drugs include oxybutynin, imidafenacin, trospium, tolterodine, glycopyrrolate, propantheline, benztropine, atropine, homatropine, tropicamide, benactyzine, biperiden, scopolamine, scopolamine butyl bromide, cyclopentolate, darifenacin, dexetimide, dicyclomine, emepronium, hexahydrosiladifenidol, octylonium, orphenadrine, oxyphenonium, pirenzepine, procyclidine, darotropium, ipratropium, tiotropium, oxitropium, quinidine, trihexyphenidyl, mivacurium, atracurium, doxacurium, cisatracurium, vecuronium, rocuronium, pancuronium, tubocurarine, gallamine, pipecuronium, trimethaphan, succinylcholine, suxamethonium, decamethonium and hexamethonium. According to the invention the anticholinergic drug is oxybutynin or a pharmaceutically acceptable salt thereof. An example of a pharmaceutically acceptable salt of oxybutynin is oxybutynin hydrochloride.

The content of the anticholinergic drug ranges from 10 mass % to 20 mass% based on the total mass of the liquid topical preparation. The content of the anticholinergic drug may range from 15 mass% to 20 mass% based on the total mass of the liquid topical preparation. The lower limit of the content of the anticholinergic drug may be 10, 12, 15 or 18 mass% based on the total mass of the liquid topical preparation.

Dicarboxylic acid ester decreases the viscosity of the liquid topical preparation, thereby suppressing "stickiness." Specific examples of the dicarboxylic acid ester include diisopropyl adipate, diethyl sebacate, diisopropyl sebacate, dimethyl succinate, dibutyl adipate, diisobutyl adipate, dioctyl adipate, dioctyl sebacate, diethyl phthalate and dibutyl phthalate. The content of the dicarboxylic acid ester may range from 2.5 mass% to 15 mass% or 5 mass% to 15 mass% based on the total mass of the liquid topical preparation. The lower limit of the content of the dicarboxylic acid ester may be 1, 2.5, 3, 3.75, 5 or 8 mass% based on the total mass of the liquid topical preparation. The upper limit of the content of the dicarboxylic acid ester may be 10, 11.25, 12 or 15 mass% based on the total mass of the liquid topical preparation. With an arbitrary combination of the lower limit and the upper limit of the content of the dicarboxylic acid ester, "stickiness" derived from the anticholinergic drug can further be reduced.

The mass ratio of the anticholinergic drug to the dicarboxylic acid ester may range from 1:0.25 to 1:0.75. The lower limit of the mass ratio of the anticholinergic drug to the dicarboxylic acid ester; that is, the lower limit of the mass of the anticholinergic drug per unit mass of the dicarboxylic acid ester may be 1:0.75, 1:0.70, 1:0.65, 1:0.6, 1:0.55 or 1:0.5. The upper limit of the mass ratio of the anticholinergic drug to the dicarboxylic acid ester; that is, the upper limit of the mass of the anticholinergic drug per unit mass of the dicarboxylic acid ester may be 1:0.05, 1:0.15, 1:0.25, 1:0.3, 1:0.33, 1:0.35 or 1:0.4. With an arbitrary combination of the lower limit and the upper limit of the mass ratio of the anticholinergic drug to the dicarboxylic acid ester, "stickiness" derived from the anticholinergic drug can further be reduced.

Water in the liquid topical preparation functions as medium for dissolving or dispersing the anticholinergic drug and the dicarboxylic acid ester as well as other components. The content of water may range from 10 mass% to 99 mass%, for example, based on the total mass of the liquid topical preparation.

The liquid topical preparation may further comprise one or more salts selected from the group consisting of lactate, tartrate, acetate and phosphate, so as to enhance the accumulation of the anticholinergic drug in skin appendages. Through enhancement of the accumulation, hyperhidrosis can be treated while suppressing side effects due to administration of the anticholinergic drug such as xerostomia. The salt may be anhydride or hydrate. Lactic acid may be either L- or D-lactic acid, or may be an arbitrary mixture thereof. Tartaric acid may be any one of L-, D-, and meso-tartaric acid, or may be an arbitrary mixture thereof. Examples of the salt include a salt with a monovalent metal such as sodium, potassium and lithium, a salt with a divalent metal such as calcium and magnesium, a salt with a trivalent metal such as aluminum, and a salt with an amine compound such as ammonia, ethylenediamine, triethylamine, diethanolamine, triethanolamine and meglumine. From the viewpoint of improving the accumulation of an anticholinergic drug in skin appendages, the salt is preferably lactate and more preferably sodium lactate.

The content of the above salt may range from, for example, 0.1 mass% to 10 mass% based on the total mass of the liquid topical preparation. The molar ratio of the anticholinergic drug to the above salt in the liquid topical preparation may be, for example, within the range of 1:0.5 to 1:2.

The liquid topical preparation may comprise, in addition to the above components, a lower alcohol, a surfactant, a preservation stabilizer, a fat and an oil, a solubilizer, a filler, a moisturizer, a pH regulating agent, an osmotic pressure regulator, a thickener, a refreshing agent, an astringent and a vasoconstrictor, for example.

The lower alcohol increases the solubility and dispersibility of the anticholinergic drug, and increases the distributivity of the anticholinergic drug into skin. Specific examples of the lower alcohol include methanol, ethanol and isopropanol. The content of the lower alcohol may range from, for example, 0 mass% to 90 mass% based on the total mass of the liquid topical preparation.

The surfactant is useful for emulsifying the anticholinergic drug in a medium such as water. Specific examples of the surfactant include a nonionic surfactant (e.g., polysorbate 20, polysorbate 80, polysorbate 60, polyoxyethylene hydrogenated castor oil 20, polyoxyethylene hydrogenated castor oil 40 and polyoxyethylene hydrogenated castor oil 60), an ionic surfactant and an amphoteric surfactant. The content of the surfactant may range from, for example, 0 mass% to 10 mass% based on the total mass of the liquid topical preparation.

Specific examples of the preservation stabilizer include paraben, isopropylmethylphenol, phenoxyethanol and thymol.

Specific examples of the fat and the oil and the solubilizer include a fatty acid and a fatty alcohol.

Specific examples of the filler include an inorganic powder (e.g., talc, montmorillonite, smectite and kaolin) and an organic powder.

Specific examples of the moisturizer include a polyhydric alcohol, saccharides, urea, a vaseline and a paraffin.

The liquid topical preparation can have a pH within the range of 4.5 to 7.5. pH determination is performed using a composite glass electrode in accordance with "2.54 pH Determination" in General Tests, Processes and Apparatus, the Japanese Pharmacopoeia, Sixteenth Edition.

The liquid topical preparation may be in a form of lotion or liniment, for example, or in a form of embrocation or spray, for example, contained in an appropriate container (for example, a spray container for spraying the liquid preparation, a container for applying the liquid preparation and an aerosol container).

The liquid topical preparation can be manufactured by mixing thoroughly the above components.

After the container is shaken as necessary to thoroughly mix the components homogeneously, the liquid topical preparation is applied to, sprinkled on or sprayed on the areas of skin where sweating should be suppressed, and is spread as needed.

### Examples

### Test example 1 - reference example

Lotions were prepared according to the compositions in Table 1, and then 500 µL of each lotion was applied to the palms of subjects (4 subjects). Each subject spread the lotion evenly over both palms by rubbing the palms together, and then 3 minutes later, gave scores according to the degree of "stickiness" based on the following 3 stages. 0: No stickiness felt
1: Stickiness felt
2: Strong stickiness felt

Based on the mean value of the scores, the degree of stickiness was evaluated based on the following 3 stages.
○: Mean value was less than 0.5
Δ: Mean value was 0.5 or more and less than 1.0
×: Mean value was 1.0 or more

**[Table 1]**

| Reference example | 1 | 2 | 3 | 4 |
|---|---|---|---|---|
| Oxybutynin hydrochloride | 8 | 10 | 15 | 20 |
| Sodium lactate | 2.3 | 2.8 | 4.3 | 5.7 |
| Ethanol | 40 | 40 | 40 | 40 |
| Purified water | 49.7 | 47.2 | 40.7 | 34.3 |
| Total | 100 | 100 | 100 | 100 |

Results are shown in Table 2. When the content of oxybutynin hydrochloride was 10 mass% or more, the subjects felt stickiness, and when the same was 15 mass% or more, the subjects felt strong stickiness.

**[Table 2]**

| Reference example | 1 | 2 | 3 | 4 |
|---|---|---|---|---|
| Mean value | 0.25 | 0.75 | 1.75 | 2 |
| Evaluation | ○ | Δ | × | × |

### Test example 2

Lotions were prepared according to the compositions in Table 3, and then the impulse value of each lotion was measured by the following method. It is indicated that the lower the impulse value, the lower the viscosity.
1) 50 µL of a lotion was placed in a 96-well plate, left to stand overnight at 32°C, and then dried.
2) A probe made of SUS (diameter: 5 mm) of a texture analyzer was brought into contact with the dried lotion.
3) The probe was ascended at the speed of 2 mm/second, the force applied thereto when the probe was moved away from the lotion was measured, and then the area under the curve was calculated as an impulse value (g·second).

**[Table 3]**

| | Comparative example | | | | | | | | | | |
|---|---|---|---|---|---|---|---|---|---|---|---|
| | 1 | 2 | 3 | 4 | 5 | 6 | 7 | 8 | 9 | 10 | 11 |
| Oxybutynin hydrochloride | 20 | 20 | 20 | 20 | 20 | 20 | 20 | 20 | 20 | 20 | 20 |
| Sodium lactate | 5.7 | 5.7 | 5.7 | 5.7 | 5.7 | 5.7 | 5.7 | 5.7 | 5.7 | 5.7 | 5.7 |
| Eucalyptus oil | | 5 | | | | | | | | | |
| Dimethicone 350 | | | 5 | | | | | | | | |
| Lauryl alcohol | | | | 5 | | | | | | | |
| Oleyl alcohol | | | | | 5 | | | | | | |
| PEG200 | | | | | | 5 | | | | | |
| PEG400 | | | | | | | 5 | | | | |
| Monolaurate PEG | | | | | | | | 5 | | | |
| Lauromacrogol | | | | | | | | | 5 | | |
| POE oleyl ether | | | | | | | | | | 5 | |
| POE cetyl ether | | | | | | | | | | | 5 |
| Ethanol | 40 | 40 | 40 | 40 | 40 | 40 | 40 | 40 | 40 | 40 | 40 |
| Purified water | 34.3 | 29.3 | 29.3 | 29.3 | 29.3 | 29.3 | 29.3 | 29.3 | 29.3 | 29.31 | 29.3 |
| Total | 100 | 100 | 100 | 100 | 100 | 100 | 100 | 100 | 100 | 100 | 100 |

| | Example | | | | | | | | | | |
|---|---|---|---|---|---|---|---|---|---|---|---|
| | 1 | 2 | 3 | 16 | 17 | 18 | | | | | |
| Oxybutynin hydrochloride | 20 | 20 | 20 | 20 | 20 | 20 | | | | | |
| Sodium lactate | 5.7 | 5.7 | 5.7 | 5.7 | 5.7 | 5.7 | | | | | |
| Diisopropyl adipate | 5 | | | | | | | | | | |
| Diethyl sebacate | | 5 | | | | | | | | | |
| Diisopropyl sebacate | | | 5 | | | | | | | | |
| Diisobutyl adipate | | | | 5 | | | | | | | |
| Dimethyl succinate | | | | | 5 | | | | | | |
| Dibutyl phthalate | | | | | | 5 | | | | | |
| Ethanol | 40 | 40 | 40 | 40 | 40 | 40 | | | | | |
| Purified water | 29.3 | 29.3 | 29.3 | 29.3 | 29.3 | 29.3 | | | | | |
| Total | 100 | 100 | 100 | 100 | 100 | 100 | | | | | |

Results are shown in Table 4. The impulse value of each lotion is the mean value of three measurements. The lotion supplemented with lauryl alcohol, oleyl alcohol, lauromacrogol, diisopropyl adipate, diethyl sebacate, diisopropyl sebacate, diisobutyl adipate, dimethyl succinate or dibutyl phthalate, when dried, had a low impulse value compared with other lotions, which confirmed the decrease in viscosity.

**[Table 4]**

| | Comparative example | | | | | | | | | | |
|---|---|---|---|---|---|---|---|---|---|---|---|
| | 1 | 2 | 3 | 4 | 5 | 6 | 7 | 8 | 9 | 10 | 11 |
| Impulse value | 6.4 | 4.8 | 8.4 | 1.4 | 1.2 | 4.9 | 5.0 | 5.3 | 1.2 | 3.6 | 3.5 |

| | Example | | | | | | | | | | |
|---|---|---|---|---|---|---|---|---|---|---|---|
| | 1 | 2 | 3 | 16 | 17 | 18 | | | | | |
| Impulse value | 0.5 | 1.5 | 1.3 | 0.91 | 0.39 | 1.3 | | | | | |

### Test example 3

Lotions were prepared according to the compositions in Table 5, and then 300 µL of each lotion was applied to the palms of subjects (3 subjects). In Comparative examples 1, 4, 5 and 9 as well as Examples 1 to 3, the same lotions as in test example 2 were used. Each subject spread the lotion evenly over both palms by rubbing the palms together, and then 3 minutes later, gave scores according to the degree of "stickiness" based on the following 4 stages.
0: No stickiness felt
1: Slight stickiness felt
2: Stickiness felt
3: Strong stickiness felt

Based on the mean value of the scores, the degree of stickiness was evaluated based on the following 5 stages.
⊚: Mean value was less than 0.1
○: Mean value was 0.1 or more and less than 1.0
Δ: Mean value was 1.0 or more and less than 2.0
×: Mean value was 2.0 or more and less than 3.0
× ×: Mean value was 3.0 or more

**[Table 5]**

| | Comparative example | | | | | | | | |
|---|---|---|---|---|---|---|---|---|---|
| | 1 | 4 | 5 | 9 | | | | | |
| Oxybutynin hydrochloride | 20 | 20 | 20 | 20 | | | | | |
| Sodium lactate | 5.7 | 5.7 | 5.7 | 5.7 | | | | | |
| Lauryl alcohol | | 5 | | | | | | | |
| Oleyl alcohol | | | 5 | | | | | | |
| Lauromacrogol | | | | 5 | | | | | |
| Ethanol | 40 | 40 | 40 | 40 | | | | | |
| Purified water | 34.3 | 343 | 34.3 | 34.3 | | | | | |
| Total | 100 | 100 | 100 | 100 | | | | | |
| Oxybutynin hydrochloride: dicarboxylic acid ester | 1:0 | 1:0 | 1:0 | 1:0 | | | | | |

| | Example | | | | | | | | |
|---|---|---|---|---|---|---|---|---|---|
| | 1 | 2 | 3 | 4 | 5 | 6 | 7 | 8 | 9 |
| Oxybutynin hydrochloride | 20 | 20 | 20 | 20 | 20 | 20 | 20 | 20 | 20 |
| Sodium lactate | 5.7 | 5.7 | 5.7 | 5.7 | 5.7 | 5.7 | 5.7 | 5.7 | 5.7 |
| Diisopropyl adipate | 5 | | | 8 | | 10 | | 15 | |
| Diethyl sebacale | | 5 | | | 8 | | 10 | | 15 |
| Diisopropyl sebacale | | | 5 | | | | | | |
| Ethanol | 40 | 40 | 40 | 40 | 40 | 40 | 40 | 40 | 40 |
| Purified water | 29.3 | 29.3 | 29.3 | 26.3 | 26.3 | 24.3 | 24.3 | 19.3 | 19.3 |
| Total | 100 | 100 | 100 | 100 | 100 | 100 | 100 | 100 | 100 |
| Oxybutynin hydrochloride: dicaboxylic acid ester | 1: 0.25 | 1: 0.25 | 1: 0.25 | 1: 0.4 | 1: 0.4 | 1: 0.5 | 1: 0.5 | 1: 0.75 | 1: 0.75 |

| | Comparative example | Example | | | Comparative example | Example | | | |
|---|---|---|---|---|---|---|---|---|---|
| | 12 | 10 | 11 | 12 | 13 | 13 | 14 | 15 | |
| Oxybutynin hydrochloride | 15 | 15 | 15 | 15 | 10 | 10 | 10 | 10 | |
| Sodium lactate | 4.28 | 4.28 | 4.28 | 4.28 | 2.85 | 2.85 | 2.85 | 2.85 | |
| Diisopropyl adipate | | 3.75 | 5 | 11.25 | | 2.5 | 5 | 7.5 | |
| Ethanol | 40 | 40 | 40 | 40 | 40 | 40 | 40 | 40 | |
| Purified water | 40.72 | 36.97 | 35.72 | 29.47 | 47.15 | 44.65 | 42.15 | 39.65 | |
| Total | 100 | 100 | 100 | 100 | 100 | 100 | 100 | 100 | |
| Oxybutynin hydrochloride: dicarboxylic acid ester | 1:0 | 1: 0.25 | 1: 0.33 | 1: 0.75 | 1:0 | 1: 0.25 | 1: 0.5 | 1: 0.75 | |

Results are shown in Table 6. The lotion supplemented with diisopropyl adipate, diethyl sebacate or diisopropyl sebacate was confirmed to have lowered stickiness compared with lotions comprising no dicarboxylic acid ester.

**[Table 6]**

| | Comparative example | | | | | | | | |
|---|---|---|---|---|---|---|---|---|---|
| | 1 | 4 | 5 | 9 | | | | | |
| Mean value | 3 | 2 | 2 | 2 | | | | | |
| Evaluation | ×× | × | × | × | | | | | |

| | Example | | | | | | | | |
|---|---|---|---|---|---|---|---|---|---|
| | 1 | 2 | 3 | 4 | 5 | 6 | 7 | 8 | 9 |
| Mean value | 1 | 0.67 | 0.33 | 0 | 0 | 0 | 0 | 0 | 0.33 |
| Evaluation | Δ | ○ | ○ | ⊚ | ⊚ | ⊚ | ⊚ | ⊚ | ○ |

| | Comparative example | Example | | | Comparative example | Example | | | |
|---|---|---|---|---|---|---|---|---|---|
| | 12 | 10 | 11 | 12 | 13 | 13 | 14 | 15 | |
| Mean value | 2.33 | 0.67 | 0.67 | 0 | 1.33 | 0.67 | 0.33 | 0 | |
| Evaluation | × | ○ | ○ | ⊚ | Δ | ○ | ○ | ⊚ | |

### Test example 4

Using the lotions of Examples 1 to 9 and Comparative example 1 in test examples 2 and 3, the preservation stability of oxybutynin was evaluated by the following method.
1) 100 µL of each lotion was measured and weighed.
2) 50 mL of mobile phase was added and mixed therewith, and then the oxybutynin concentration of each lotion was measured by high-performance liquid chromatography (HPLC). HPLC conditions are as follows.
   Mobile phase: 0.1 w/w% aqueous phosphoric acid solution (containing 0.5 w/v% sodium dodecyl sulfate):acetonitrile = 45:55(v/v)
   Flow rate: 1.5 mL/minute
   Column: TSK gel ODS-80Ts (Tosoh Corporation)
   Retention time: 10 minutes
3) After the lotion was left to stand at 60°C for 2 weeks, the oxybutynin content (%) per solution weight, relative to the theoretical value, was compared with the initial oxybutynin content (%) relative to the theoretical value. When the oxybutynin content (%) after the lotion was left to stand at 60°C for 2 weeks was 97.5% or more of the initial oxybutynin content (%), the preservation stability of the oxybutynin was evaluated as good.

Results are shown in Table 7. In the lotion supplemented with diisopropyl adipate, diethyl sebacate or diisopropyl sebacate, oxybutynin hydrochloride was preserved well even after the lotion was left to stand under an environment at 60°C for 2 weeks. Moreover, in Comparative example 1 wherein no dicarboxylic acid ester was added, oxybutynin hydrochloride was preserved well.

**[Table 7]**

| | | Initial value | After left to stand | After left to stand/initial value (%) |
|---|---|---|---|---|
| Example | 1 | 101.1 | 101.3 | 100.2 |
| | 2 | 102.2 | 100.6 | 98.4 |
| | 3 | 101.2 | 100.4 | 99.1 |
| | 4 | 100.3 | 100.2 | 99.9 |
| | 5 | 100.3 | 99.8 | 99.5 |
| | 6 | 100.4 | 100.0 | 99.6 |
| | 7 | 101.3 | 100.9 | 99.6 |
| | 8 | 102.1 | 100.9 | 98.8 |
| | 9 | 101.7 | 100.7 | 99.0 |
| Comparative example | 1 | 100.8 | 99.7 | 98.9 |

### Test example 5

Using the lotions of Comparative example 1 and Example 4 in test example 3, the skin permeability of oxybutynin was determined by the following method.
1) 5 µL of each lotion was applied to an area of 3 cm² on the dermatomed human skin surface.
2) After several seconds of drying, the skin was set in Franz Cell so that the skin dermis side was the receptor layer side. As the receptor layer, physiological saline was used. At the time points, 4, 8, 12, 16, 20 and 24 hours after setting of the skin, the receptor solution was sampled. To 0.5 mL of the sampled solution, 0.5 mL of acetonitrile was added, the mixture was stirred, and then centrifugation was performed for deproteinization, thereby preparing a test solution.
3) The oxybutynin concentration in the test solution was measured by HPLC under the same conditions as those in test example 4.
4) The skin permeation rate of oxybutynin per hour was calculated from the thus obtained measurement, and the maximum value was designated as Jmax (µg/cm²/h). Furthermore, the cumulative amount (µg/cm²) permeated in 24 hours was found.

Results are shown in Table 8. The lotion supplemented with diisopropyl adipate exerted the same degree of skin permeability as that of lotions comprising no dicarboxylic acid ester.

**[Table 8]**

| | Jₘₐₓ (ug/cm²/h) | Cumulative amount 24h (ug/cm²) | Availability (%) |
|---|---|---|---|
| Comparative example 1 | 0.58 | 9.4 | 2.8 |
| Example 4 | 0.50 | 8.4 | 2.5 |

### Test example 6

Lotions were prepared according to the compositions in Table 9, and visually confirmed for the state of dissolution. Furthermore, the lotions were applied to porcine skin, and then the amounts of oxybutynin accumulated in hair follicles were measured by the following method.
1) 20 µL of a lotion was applied to 5 cm² of lightly shaved porcine skin. Number of pigs: n = 3.
2) After 6 hours, the skin surface was cleaned with ethanol for disinfection, and washed with a stream of phosphate buffer, thereby removing oxybutynin that had adhered to the skin surface.
3) A hair follicle portion of 20 hairs was collected from the skin.
4) Oxybutynin was extracted from the hair follicles using 1 mL of an extracting liquid. As the extracting liquid, the mobile phase of test example 4 was used.
5) Oxybutynin concentration was measured by HPLC. HPLC conditions are the same as those in test example 4.

**[Table 9]**

| Composition | 1 | 2 | 3 | 4 | 5 | 6 | 7 | 8 | 9 | 10 |
|---|---|---|---|---|---|---|---|---|---|---|
| Oxybutynin | 4.54 | - | | | | | | | | |
| Oxybutynin hydrochloride | - | 5 | 5 | 5 | 5 | 5 | 5 | 5 | 5 | 5 |
| Sodium hydroxide | | | 0.51 | | | | | | | |
| Disodium hydrogenphosphate | | | | 0.9 | | | | | | |
| Sodium lactate | | | | | 1.42 | | | | | |
| Sodium acetate | | | | | | 1.04 | | | | |
| Disodium fumarate | | | | | | | 1.02 | | | |
| Trisodium citrate | | | | | | | | 1.09 | | |
| Sodium benzoate | | | | | | | | | 1.83 | |
| Disodium tartrate-dihydrate | | | | | | | | | | 2.92 |
| Ethanol | 40 | 40 | 40 | 40 | 40 | 40 | 40 | 40 | 40 | 40 |
| Others | 0.2 | 0.2 | 0.2 | 0.2 | 0.2 | 0.2 | 0.2 | 0.2 | 0.2 | 0.2 |
| Purified water | 55.26 | 54.8 | 52.49 | 53.9 | 53.38 | 58.76 | 53.78 | 53.71 | 52.97 | 51.88 |
| Total | 100 | 100 | 100 | 100 | 100 | 100 | 100 | 100 | 100 | 100 |

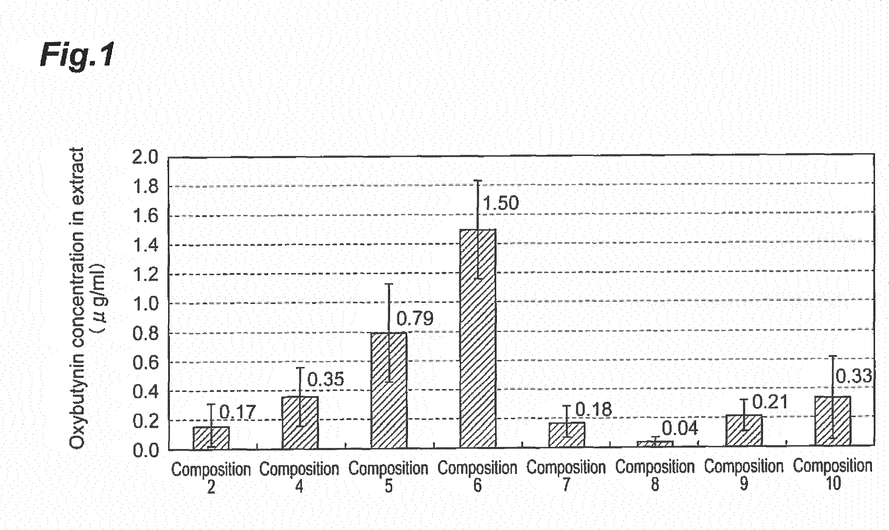

Results are shown in Table 10 and Figure 1. With the lotion comprising phosphate, lactate, acetate or tartrate, accumulation of oxybutynin in hair follicles was high compared to lotions comprising none of these salts.

**[Table 10]**

| Composition | 1 | 2 | 3 | 4 | 5 | 6 | 7 | 8 | 9 | 10 |
|---|---|---|---|---|---|---|---|---|---|---|
| State of dissolution | × | × | × | ○ | ○ | ○ | ○ | ○ | ○ | ○ |

### Test example 7 - reference example

Lotions were prepared according to the compositions in Table 11. The lotions were determined for the effect of suppressing sweating by a pilocarpine-induced sweat test. Moreover, in a manner similar to that in test example 6, the lotions were applied to porcine skin, and then oxybutynin concentrations were measured.

The pilocarpine-induced sweat test was conducted by the following method.
1) A lotion was diluted 12-fold with a 40 mass% aqueous ethanol solution.
2) 10 µL or 15 µL of the lotion was applied to about 0.5 cm² of a mouse footpad. Number of mice: n = 5 to 6.
3) After 4 hours, iodine and a starch solution were applied to the footpad under anesthesia.
4) Pilocarpine was intradermally administered at 5 µg/foot.
5) After 5 minutes, the number of black spots resulting from the iodostarch reaction was counted.

**[Table 11]**

| Composition | 11 | 12 | 13 | 14 | 15 |
|---|---|---|---|---|---|
| Oxybutynin hydrochloride | 0 | 1 | 2.5 | 5 | 10 |
| Lactic acid | 2.29 | | | | |
| Sodium chloride | 1.48 | | | | |
| Sodium lactate | | 0.28 | 0.71 | 1.42 | 2.84 |
| Ethanol | 40 | 40 | 40 | 40 | 40 |
| Others | 2 | 2 | 2 | 2 | 2 |
| Purified water | 54.23 | 56.72 | 54.79 | 51.58 | 45.16 |
| Total | 100 | 100 | 100 | 100 | 100 |

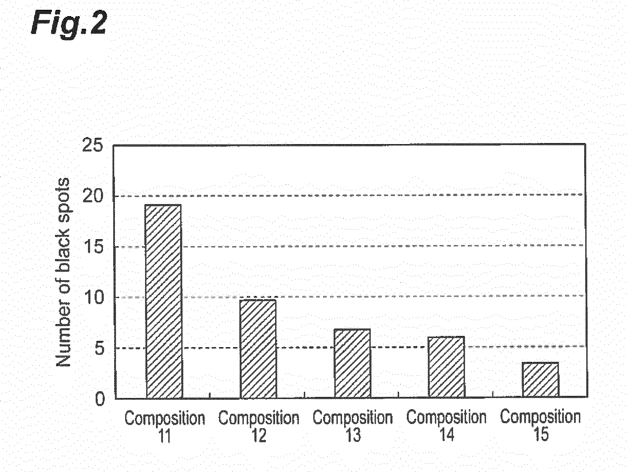

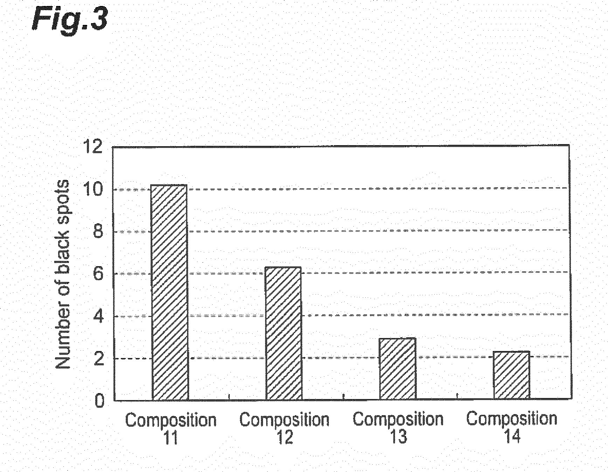

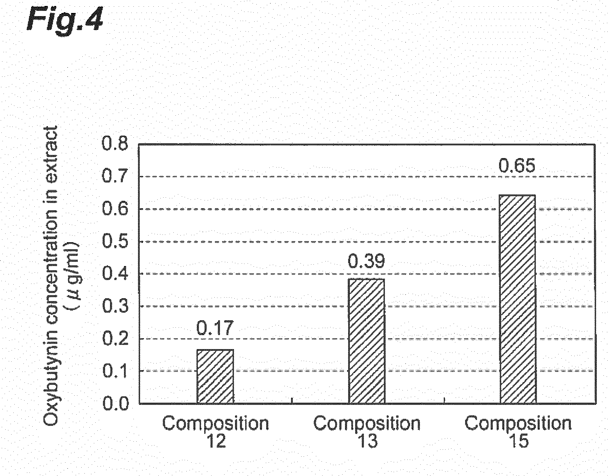

Results are shown in Figure 2 to Figure 4. Figure 2 shows the results of the pilocarpine-induced sweat test when the amount of each lotion applied was 10 µL, and Figure 3 shows the results of the pilocarpine-induced sweat test when the amount of each lotion applied was 15 µL. It was confirmed that the lotions' effect of suppressing sweating was oxybutynin concentration-dependent. It was also confirmed that the amounts of oxybutynin accumulated in hair follicles were oxybutynin concentration-dependent.

## Claims

1. A liquid topical preparation, comprising:
water;
oxybutynin or a pharmaceutically acceptable salt thereof; and
a dicarboxylic acid ester,
wherein the dicarboxylic acid ester is one or more compounds selected from the group consisting of diisopropyl adipate, diethyl sebacate, diisopropyl sebacate, diisobutyl adipate, dimethyl succinate and dibutyl phthalate, and
wherein a content of oxybutynin or a pharmaceutically acceptable salt thereof ranges from 10 mass% to 20 mass% based on a total mass of the liquid topical preparation.

2. The liquid topical preparation according to claim 1, wherein the content of oxybutynin or a pharmaceutically acceptable salt thereof ranges from 15 mass% to 20 mass% based on the total mass of the liquid topical preparation.

3. The liquid topical preparation according to claim 1 or 2, wherein a mass ratio of oxybutynin or a pharmaceutically acceptable salt thereof to the dicarboxylic acid ester ranges from 1:0.25 to 1:0.75.

4. The liquid topical preparation according to any one of claims 1 to 3, wherein a content of the dicarboxylic acid ester ranges from 2.5 mass% to 15 mass% based on the total mass of the liquid topical preparation.

5. The liquid topical preparation according to any one of claims 1 to 4, further comprising one or more salts selected from the group consisting of lactate, tartrate, acetate and phosphate.

6. The liquid topical preparation according to claim 5, wherein the salt is sodium lactate.

7. The liquid topical preparation according to any one of claims 1 to 6 in a form of lotion.

8. The liquid topical preparation according to any one of claims 1 to 7 for use in treating hyperhidrosis.

## Patentansprüche

1. Flüssiges topisches Präparat, bestehend aus:
Wasser;
Oxybutynin oder eine, pharmazeutisch verträglichen Salz davon; und
einem Dicarbonsäureester,
wobei der Dicarbonsäureester eine oder mehrere Verbindungen ist, ausgewählt aus der Gruppe bestehend aus Diisopropyladipat, Diethylsebacat, Diisopropylsebacat, Diisobutyladipat, Dimethylsuccinat und Dibutylphthalat, und
wobei der Gehalt an Oxybutynin oder einem pharmazeutisch verträglichen Salz davon im Bereich von 10 Massen-% bis 20 Massen-%, bezogen auf die Gesamtmasse der flüssigen topischen Zubereitung, liegt.

2. Flüssige topische Zubereitung nach Anspruch 1, wobei der Gehalt an Oxybutynin oder einem pharmazeutisch verträglichen Salz davon im Bereich von 15 Massen-% bis 20 Massen-%, bezogen auf die Gesamtmasse der flüssigen topischen Zubereitung, liegt.

3. Flüssige topische Zubereitung nach Anspruch 1 oder 2, wobei das Massenverhältnis von Oxybutynin oder einem pharmazeutisch verträglichen Salz davon zu dem Dicarbonsäureester im Bereich von 1:0,25 bis 1:0,75 liegt.

4. Flüssige topische Zubereitung nach einem der Ansprüche 1 bis 3, wobei der Gehalt des Dicarbonsäureesters im Bereich von 2,5 Massen-% bis 15 Massen-%, bezogen auf die Gesamtmasse der flüssigen topischen Zubereitung, liegt.

5. Flüssige topische Zubereitung nach einem der Ansprüche 1 bis 4, die ferner ein oder mehrere Salze, ausgewählt aus der Gruppe bestehend aus Lactat, Tartrat, Acetat und Phosphat, enthält.

6. Flüssige topische Zubereitung nach Anspruch 5, wobei das Salz Natriumlactat ist.

7. Flüssige topische Zubereitung nach einem der Ansprüche 1 bis 6 in Form einer Lotion.

8. Flüssige topische Zubereitung nach einem der Ansprüche 1 bis 7 zur Verwendung bei der Behandlung von Hyperhidrose.

## Revendications

1. Préparation topique liquide, comprenant :
de l'eau ;
de l'oxybutynine ou son sel pharmaceutiquement acceptable ; et
un ester d'acide dicarboxylique,
l'ester d'acide dicarboxylique étant un ou plusieurs composés sélectionnés dans le groupe constitué de l'adipate de diisopropyle, du sébaçate de diéthyle, du sébaçate de diisopropyle, de l'adipate de diisobutyle, du succinate de diméthyle et du phtalate de dibutyle, et
une teneur de l'oxybutynine ou son sel pharmaceutiquement acceptable s'étendant de 10 % en masse à 20 % en masse sur la base d'une masse totale de la préparation topique liquide.

2. Préparation topique liquide selon la revendication 1, la teneur de l'oxybutynine ou son sel pharmaceutiquement acceptable s'étendant de 15 % en masse à 20 % en masse sur la base de la masse totale de la préparation topique liquide.

3. Préparation topique liquide selon la revendication 1 ou 2, un rapport en masse de l'oxybutynine ou son sel pharmaceutiquement acceptable à l'ester d'acide dicarboxylique s'étendant de 1:0,25 à 1:0,75.

4. Préparation topique liquide selon l'une quelconque des revendications 1 à 3, une teneur de l'ester d'acide dicarboxylique s'étendant de 2,5 % en masse à 15 % en masse sur la base de la masse totale de la préparation topique liquide.

5. Préparation topique liquide selon l'une quelconque des revendications 1 à 4, comprenant en outre un ou plusieurs sels sélectionnés dans le groupe constitué du lactate, tartrate, acétate et phosphate.

6. Préparation topique liquide selon la revendication 5, le sel étant le lactate de sodium.

7. Préparation topique liquide selon l'une quelconque des revendications 1 à 6 sous la forme d'une lotion.

8. Préparation topique liquide selon l'une quelconque des revendications 1 à 7 pour l'utilisation dans le traitement de l'hyperhidrose.
